# EUROPEAN PATENT APPLICATION

(11) **EP 1 462 102 A1**
(43) Date of publication of application: **29.09.2004**
(21) Application number: 04005580.8
(22) Date of filing: 09.03.2004
(51) Int. Cl.: A61K 31/192, A61K 38/43, A01K 67/027

(54) **A method for promoting gonadal growth in an animal**

(30) Priority: 27.03.2003 JP 2003088231
(71) Applicant: NAGOYA UNIVERSITY, Chikusa-ku Nagoya City Aichi Prefecture (JP)
(72) Inventor: Yoshimura, Takashi, Hirabari-shukusha E542, Nagoya City Aichi Pref. (JP); Ebihara, Shizufumi, Nagoya City Aichi Pref. (JP)
(74) Representative: Wibbelmann, Jobst, Dr., Dipl.-Chem.

(57) **Abstract**

The present invention provided a method for promoting gonadal growth in an animal, comprising administration of thyroid hormone or its derivatives having thyroid hormone-like activity to the animal. Furthermore, this invention also provided a method for promoting gonadal growth in an animal, comprising introduction of a gene encoding type II deiodinase into the animal, and a transformed animal introduced with a gene encoding type II deiodinase into the animal. The method of the present invention provides a new method for promoting gonadal growth in an animal, through elucidation on the molecular mechanism of photoperiodism (photoperiodic time measurement) in birds.

## Description

### BACKGROUND OF THE INVENTION

### 1. Field of the Invention

The present invention relates to a method for promoting gonadal growth in an animal, comprising administration of thyroid hormone or its derivatives having thyroid hormone-like activity to the animal. Furthermore, this invention relates to a method for promoting gonadal growth in an animal, comprising introduction of a gene encoding type II deiodinase into the animal. Furthermore, this invention relates to a transformed animal introduced with a gene encoding type II deiodinase into the animal.

### 2. Prior Art

In birds, it is recognized that secretions of gonadotropin releasing hormone (GnRH) in hypothalamus and luteinizing hormone (LH) in pituitary gland are regulated by stimulation of visible light. This phenomenon, which is known to be induced by light stimulation, is called photoperiodism (photoperiodic time measurement, refer to PTM). It is considered that the region responsible for control of PTM exists in central nervous system (hypothalamus).

As gonadotropin affects the function of ovarian or testis, reproduction of poultries receives regulation by PTM as described above. Using such property, in raising poultries such as Japanese quails and chickens, regulation of light illumination has been utilized to control egg collection. For example, in chickens, egg productivity has been improved utilizing long day condition and short day condition, that is, chickens are placed under long day condition of 14 to 16 hours of light to produce eggs, and then placed under short day condition of less than 12 hours of light to stop producing eggs.

Furthermore, reproduction of livestocks such as horses, sheep and goats, as well as poultries, receives regulation by photoperiodism, thus it is known that they exhibit seasonal reproduction. Specifically, horse reproduction is mainly concentrated in spring season because elongated day length activates function of female ovary. Meanwhile, sheep reproduction is mainly concentrated in autumn season because shortened day length activates function of female ovary. Therefore, horse is referred to a "long-day reproduction animal" and sheep is referred to a "short-day reproduction animal".

Sheep is a short-day reproducing animal, therefore, reproductivity of sheep is regulated by the method of making an artificial condition that mimics the short-day condition. This method is achieved by administration of melatonin, which is a hormone released from pineal gland during night, and such method has been applied industrially. However, the method utilizing melatonin is effective in only some short-day reproduction animals, while it is ineffective in long-day reproduction animals or birds such as poultry, which remained as a problem to be solved.

### SUMMARY OF THE INVENTION

As a technique for regulation of poultry reproduction, established technique except for above-mentioned light control method has not been known until now. Thus, there has been demand on a new method for regulation of reproduction in various poultries and livestocks. Accordingly, an object of the present invention is to provide a new technique applicable for regulation of poultry reproduction, by analyzing the mechanism responsible for PTM regulation in central nervous system. If such a new technique can be provided in this invention, it will be useful for livestock industry. In addition, it will be helpful for saving species under the crisis of annihilation.

To solve the object as described above, the present invention provides a method for promoting gonadal growth in an animal, comprising administration of thyroid hormone or its derivatives having thyroid hormone-like activity to the animal. Furthermore, this invention relates to a method for promoting gonadal growth in an animal, comprising introduction of a gene encoding type II deiodinase into the animal. Furthermore, this invention relates to a transformed animal introduced with a gene encoding type II deiodinase into the animal.

In the following, this invention is explained indetail, however, these detailed explanation and the examples do not intend to restrict or limit the effective range of this invention.

### BRIEF DESCRIPTION OF THE DRAWINGS

Fig. 1 is a figure showing structures of T₄ and T₃.
Fig. 2 is a figure showing the metabolic pathway of T₄ by deiodinase.
Fig. 3 is a figure showing the brain region used to dissect the gene.
Fig. 4 is a photograph showing the photo-induced expression of D2 gene in nucleus hypothalamus posterior medialis (NHPM).
Fig. 5 is a graph showing the effects of light pulse and light length on the expression of the D2 gene in the NHPM.
Fig. 6 is a photograph showing the expression of D2 gene, induced by long-day stimulation, in the IN and the ME.
Fig. 7 is a graph showing the effects of the light pulse and light length on the expression of D2 in the IN and the ME.
Fig. 8 is a graph showing plasma contents of T₃ and T₄ in the short-day and long-day groups (Fig. 8a), and T₃ (Fig. 8b) and T₄ (Fig. 8c) contents in MBH, SGC (stratum griseum centrale) and Cb (cerebellum).
Fig. 9 is a photograph showing the expression of thyroid hormone receptor genes (TRα, β and RXα) in the IN and the ME.
Fig. 10 is a graph showing testicular growth by administration T₃ and T₄, and the effect of IOP on the testicular growth.

### DETAILED DESCRIPTION OF THE INVENTION

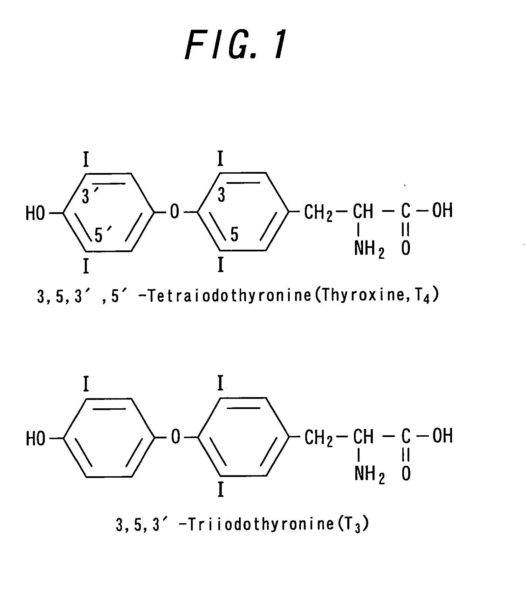

Thyroxine (refer to T₄) and 3,5,3'-triiodothyronine (refer to T₃) are major hormones secreted from thyroid (thyroid hormones) and they are involved in calorigenic action of a living body. T₃ is not only secreted from thyroid, but it is also produced by deiodinase peripherally. Moreover, reverse triiodothyronine (3,5,5'-triiodothyronine, refer to RT₃) is also known to exist, as a molecule similar to T₃ with difference from T₃ in the iodine binding position. The hormonal activity of T₃ is stronger than that of T₄, while RT₃ is inactive. The structures of T₄ and T₃ are shown in the following Fig. 1.

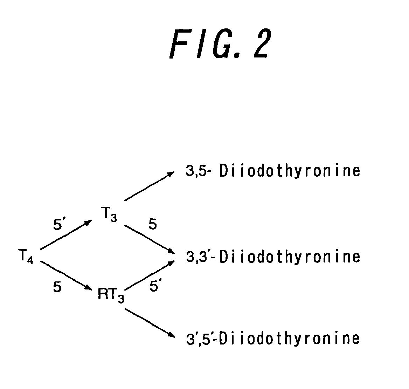

Deiodinase as described above is an enzyme exhibiting wide range distribution, not only in thyroid, but also in liver, kidney, muscle, pituitary gland and so on. Deiodinase is known to catalyze deiodination reaction of T₄ to produce T₃. 5'-Deiodinase and 5-deiodinase are involved in deiodination reaction of T₄, and they catalyze production of T₃ and RT₃, respectively.
Produced T₃ and RT₃ are converted to various diiodothyronines. The metabolic pathway of T₄ by deiodinase is shown in Fig. 2.

5'-Deiodinase is known to include three types of enzymes, namely type I deiodinase, type II deiodinase, and type III deiodinase. The type I deiodinase exists in microsome of liver and kidney and catalyzes conversion of T₄ to T₃ by deiodising reaction at the outer ring of T₄ and conversion of RT₃ to 3,3'-diiodothyronine. The action of type II deiodinase is similar to type I deiodinase and this enzyme exists in brain, pituitary gland and brown fat. Moreover, type III deiodinase effects only to the inner ring of T₄. and this enzyme exists in placenta and brain.

The present inventors investigated on the molecular mechanism of photoperiodism regulating reproduction of birds, as described in the following examples. Specifically, genes exhibiting induced expression in response to light stimulation was analyzed in Japanese quails (*Coturnix coturnix japonica*), using the technique of differential subtractive hybridization. In result, it was found that the expression of type II deiodinase (refer to D2) gene was induced by light in medial basal hypothalamus (MBH). Accordingly, the light-induced expression of D2 is assumed to be involved in PTM regulation.

As described in detail in the above description, type II deiodinase catalyzes peripheral deiodising reaction of T₄ to produce T₃. Accordingly, the inventors considered the possibility that the enzyme product of D2 might be involved in reproduction of birds through regulation of PTM. Therefore, the inventors administrated T₃ into cerebroventricle of Japanese quail, which result in testicular growth of the Japanese quail. This finding shows that D2, existing in central nervous system, causes conversion of T₄ to T₃, and the produced T₃ promotes gonadal growth. Accordingly, the present invention provides a new method for promoting gonadal growth of an animal comprising administration of thyroid hormone or its derivatives having thyroid hormone-like activity, through elucidation of molecular mechanism of PTM in birds.

In the present specification, "thyroid hormone or its derivatives having thyroid hormone-like activity" includes T₃ and T₄, which are the major thyroid hormones, in addition to their derivatives having thyroid hormone-like activity. As a concrete example of such derivative, TETRAC (3,5,3',5'-tetraiodothyroacetic acid) may be cited, however, such derivative is not to be limited to above example. They may also include other compounds having activity similar to T₃.

In the aspect of the root of administration, the most preferred embodiment is direct administration of T₃ to central nervous system, particularly direct intracerebroventricular administration. Because of low activity of T₄, it is estimated that no effect or only very weak effect of T₄ would be obtained when T₄ is administrated to central nervous system. However, when T₄ is administrated peripherally by the method of intravenous administration or oral administration etc, major part of the administrated T₄ would be metabolized in the blood to be converted into T₃. Accordingly, when T₄ is administrated peripherally, it is expected that T₃, produced in the blood, may act in central nervous system. Therefore, peripheral administration of T₄ is also one preferred embodiment of the present invention.

Meanwhile, when T₃ is administrated peripherally, it is expected that administrated T₃ may be metabolically converted to compounds such as 3,3'-diiodothyronine (3,3'-T₂) in blood, and then T₃ may be inactivated. Considering it, in the case of peripheral administration, administration of T₄, which is a precursor of T₃, may be more preferable than administration of T₃. When T₃ is administrated peripherally, it would be necessary to prevent T₃ from receiving metabolism in blood, by the means adopting elaborated dosage form, for example. In view of the above teaching, when thyroid hormone or its derivatives having thyroid hormone-like activity is administrated for the purpose of the present invention, various root of administration can be selected, including not only intracerebroventricular administration, but also oral, intravenous, intraarterial, intraperitoneal transdermal and mucomembranous administration.

In the following examples, the inventors obtained the greatest effect by intracerebroventricular administration of 0.3 ng of T₃ per day to Japanese quail. In the present invention, the dose of T₃ to be administrated is 1 pg to 10 µg per day, preferably 10 pg to 1 µg, more preferably 0.1 to 100 ng. However, the dose of T₃ to be administrated in the present invention is not limited within the range as described above. The optimum dose may be selected considering the compound to be administrated, species or size of the target animal to be administrated and the root of administration, for the purpose to obtain the effect aimed in this invention.

Moreover, expression of D2 can be increased in an animal, by introduction of a gene encoding D2 and over-expressing it in the animal. If D2 is over-expressed in the animal body, particularly in central nervous system, the level of T₃ would increase in the animal, as T₃ is the product of the enzyme reaction. Accordingly, gonadal growth can be promoted in an animal by producing transformed animal exhibiting hypothalamic expression of the D2 gene constitutively.

Animal species to be transformed in the present invention is not particularly limited and gonadal growth can be promoted in various animals including birds and mammals, by introducing a gene encoding D2. Above all, utilizing poultries such as quail, chicken and turkey may be particularly preferable as one embodiment of the present invention. Incidentally, production of transformants in birds has been already reported, for example, in A.J. Harvey *et al.,* "Expression of exogenous protein in the egg white of transgenic chickens" Nature biotechnology, 2002, (19), 396-399.

Furthermore, the method of the present invention may be also applicable to livestocks that belongs to mammals, such as cattle, sheep, pigs, horses and goats. A transformed animal having high reproductivity can be produced, by introducing the D2 gene into these livestocks or poultries. Accordingly, the method of the present invention may greatly contribute to development of animal industry.

The animal to be administrated T₃ may be male or female, and not to be limited to either sexuality. In the following examples, it is demonstrated that the testis of males are grown by administration of T₃. The mechanism responsible for gonadal growth is assumed to be in common between males and females, and the gonadal growth is assumed to be regulated by gonadotropin releasing hormone (GnRH) released from hypothalamus. The molecular mechanism responsible for measurement of day length is assumed to be in common between males and females, regardless of sex difference. Therefore, administration of T₃ would cause ovarian growth in females, not only cause testicular growth in males.

### EXAMPLE

### (Dissection of a gene, which is responsible for PTM)

To analyze genes responsible for the regulation of the PTM in birds, the inventors carried out differential subtractive hybridization analysis. Forty male Japanese quails (*Coturnix coturnix japonica*) were raised under short-day condition until 8 week-old. Herein, the short-day condition means the condition of 8 hours light length and 16 hours dark length, while the long day condition means the condition of 16 hours light length and 8 hours dark length. Twenty animals were exposed to 1 hr light pulse at zeitgeber time (ZT) 14, while the other twenty animals were kept in darkness.

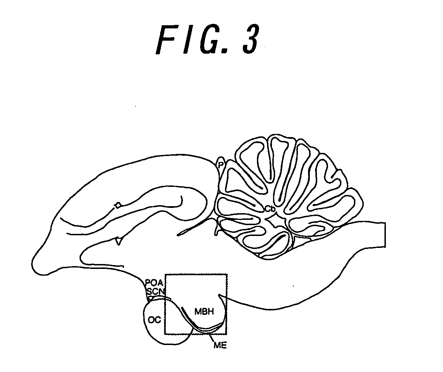

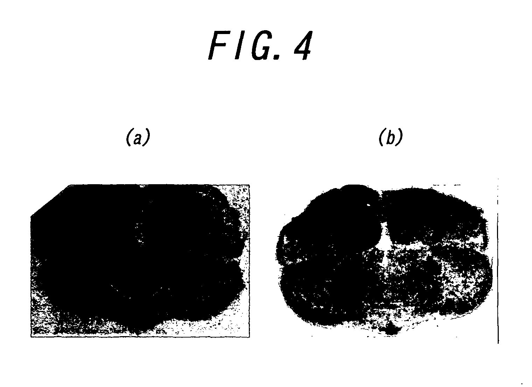

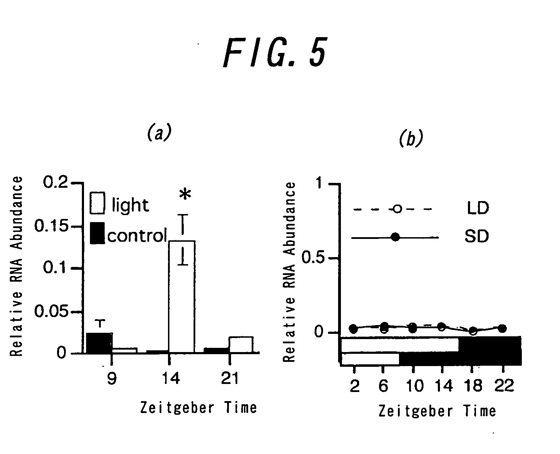

One hour after light pulse (ZT16), both groups of animals were killed by decapitation to avoid acute change in gene expression and the medial basal hypothalamus (MBH) was punched out from 3 mm brain slices. Fig. 3 shows the brain region used to dissect a gene by differential subtractive hybridization. In Fig. 3, MBH represents medial basal hypothalamus, ME represents median eminence, OC represents optic chiasm, POA represents preoptic area, SCN represents suprachiasmatic nucleus, P represents pineal gland, and Cb represents cerebellum.

Total RNA was extracted and poly (A)⁺ RNA was purified. Differential subtractive hybridization analysis was carried out according to the manufacturer's instruction (PCR-Select cDNA subtraction kit, Clontech). One hundred and fifty clones were sequenced, and expression of these genes was verified using *in situ* hybridization.

Fig. 4a is a photograph showing light-induced expression of D2 in the nucleus hypothalamus posterior medialis (NHPM). Arrows represent expression of D2. Further, Fig. 4b is a photograph of control animal, which was not given light pulse. One hour light pulse was given to the animals at ZT14 and brain was collected one hour after light pulse. Moreover, Fig. 5a is a graph showing D2 expression at various phases in the NHPM. ZT14 is within the photoinducible phase, and ZT9 and ZT21 are out of photoinducible phase.
Asterisk shows significant difference in *P*<0.01.

In result, induction of D2 gene by light pulse was found at photoinducible phase (ZT 14) (Fig. 4a, b). The inventors then examined the effect of light exposure at out of photoinducible phase (ZT 9 and 21). However, light-induced expression of D2 was not observed at ZT 9 and 21, which indicates that light induction of D2 was specific to photoinducible phase (Fig. 5a).

To explore the expression profiles of D2 gene under short days and long days, further examination of *in situ* hybridization was performed. Fig. 5b shows temporal expression profiles of D2 gene under long days (LD) and short days (SD). No significant difference was observed between two groups. Expression of D2 was almost undetectable at any time of day in both short days and long days.

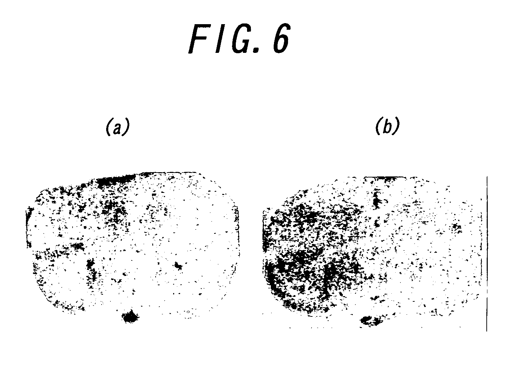

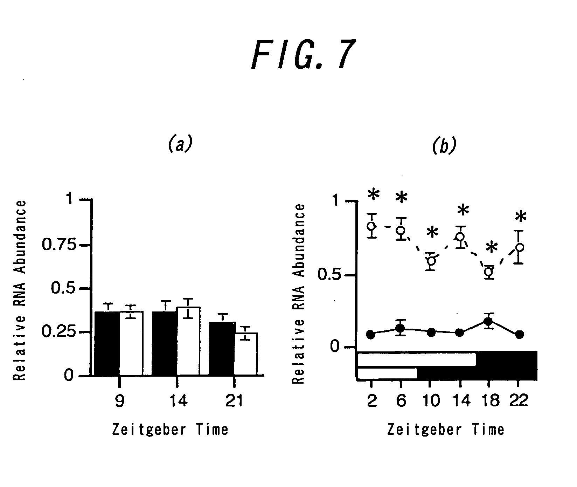

Moreover, Fig. 6 shows photographs of the ventral infundibular nucleus (IN) and the median eminence (ME) of animals, kept under short-day and long-day conditions. Fig. 6a is a photograph showing expression of D2 induced by long-day stimulation in the ventral IN and the ME, and Fig. 6b is a photograph of control animals kept under short-day condition. In result, expression of D2 was also observed in the ventral IN and the ME (Fig. 6a, b). Therefore, the inventors examined the effect of light pulse on D2 expression (Fig. 7a) and temporal expression profiles of D2 gene in these regions (Fig. 7b). In result, weak expression was observed in short days and strong expression was observed in long days in these regions.

According to the results as described above, it is found that expression of D2 is induced by light. However, the expression profiles were different among each brain region. Acute induction was observed in NHPM, but its expression was not observed in the continuous long photoperiod.
In contrast, D2 expression in the ventral IN and the ME was upregulated in long photoperiod, but acute induction was not observed in these regions.

### (Content and target site of thyroid hormone)

D2 is an enzyme, which converts thyroxine (T₄) to 3,5,3'-triiodotyronine (T₃), and is primarily responsible for thyroid hormone action. D2 plays an essential role in the local control of brain T₃, through mechanisms that operate under various situations to keep T₃ concentrations within a narrow range. The inventors examined T₃ and T₄ contents in the punched out medial basal hypothalamus (MBH: MBH of 10 animals were pooled in each group) under short days and long days.

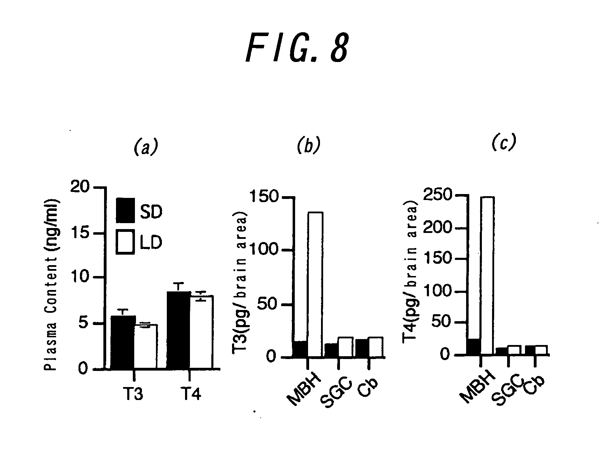

Plasma contents of T₃ and T₄ in short-day group and long-day group are shown (Fig. 8a). Moreover, T₃ (Fig. 8b) and T₄ (Fig. 8c) contents in the MBH, the SGC (stratum griseum centrale) and the Cb (cerebellum) were examined. In result, although plasma contents of T₃ and T₄ were not different between short-day and long-day groups (Fig. 8a), T₃ and T₄ contents in the MBH were about 10 fold higher in the long-day animals than in the short-day animals (Fig. 8b, c). However, this difference was not observed in other parts of brain such as SGC and Cb (Fig. 8b, c).

According to Fig. 8, although it was confirmed that T₃ content of the MBH in long-day animals is increased by D2, such difference was not observed in the serum and other parts of brain. D2 catalyzes the intracellular deiodination of the prohormone T₄ to the active T₃, indicating that D2 acts as a gate keeper to thyroid hormone action and modulates the local availability of T₃.
In addition, T₄ content was also increased in the MBH of long day animals. It seems that increased T₄ uptake helps D2 to generate more T₃ in this region under long days.

To explore the target site of locally generated T₃ to act, expression of thyroid hormone receptor (TR) genes (TRα, β, β2, RXRα, γ) was examined.

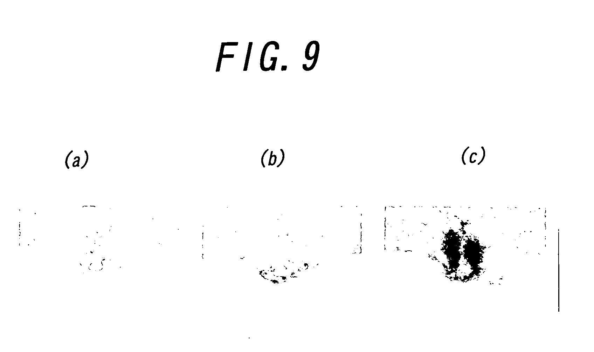

Fig. 9 shows expression of TRα gene (Fig. 9a), TRβ gene (Fig. 9b) and RXRα gene (Fig. 9c) in the IN and the ME. In result, weak expression of TRα, β genes and strong expression of RXRα gene was observed in the IN and the ME (Fig. 9a, b, c), while that of TRβ2 and RXRγ was undetectable. Expression of TRα, β and RXRα was observed all day long both under short days and long days, and did not show rhythmic expression. These results indicate that locally generated T₃ acts on the IN and the ME.

### (Gonadal growth by administration of T₃)

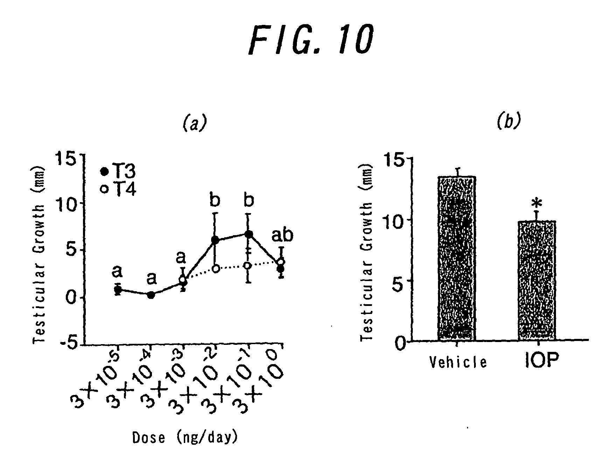

To assess directly whether T3 mediates photoperiodic response of gonads, the inventors studied the effect of intracerebroventricular (i.c.v.) infusion of T₃ on gonadal growth. Vehicle and several doses of T₃ and T₄ were infused to third ventricle by osmotic mini pump and testicular size was measured before and after the infusion. Fig. 10a shows testicular growth by administration of T₃ (closed circle) and T₄ (open circle). T₃ infusion mimics testicular growth with dose dependent manner even though animals were kept under short-day condition (light length: 8 hrs, dark length: 16 hrs) (p=0.0128, one-way ANOVA, F (5,18)=4.008), while T₄ infusion has little effect (p=0.8111, one-way ANOVA, F (3,16)=0.32). However, testicular growth was not observed in the largest dose of T₃. Iopanoic acid (IOP) is known to inhibit the conversion of T₄ to T₃. Therefore, the inventors furthermore examined the effect of IOP infusion under long-day condition (Fig. 10b). Fig. 10b shows that IOP can reduce testicular growth in long-day condition (p<0.05).

Follett *et al.* showed that peripheral injection of pharmacological dose of thyroid hormone (T₃ and T₄) can mimic photoperiodically induced gonadotropin secretion and gonadal growth (refer to Follett, B. K. *et al.,* "Acute effect of thyroid hormones in mimicking photoperiodically induced release of gonadotropins in Japanese quail" *J. Comp. Physiol. B* **157**, 837-843 (1988) and Follett, B. K. *et al.,* "Thyroxine can mimic photoperiodically induced gonadal growth in Japanese quail" *J. Comp. Physiol. B* **157**, 829-835 (1988)). In their study, however, T₄ was more effective than T₃ to mimic gonadotropin release. Their report seems to be contradictory to the inventors' result.

However, about 1/3, and 45% of the T₄ has is known to be converted to T₃ and reverse T₃ (RT₃) in the blood, respectively. Furthermore, T₃ is converted to 3, 3'-diiodothyronine (3,3'-T₂) in the blood. Therefore, it is considered that T₄, administrated from periphery, acts as T₃ in the central nervous system. In addition, the inventors have shown that IOP of D2 inhibitor reduces testicular growth under long days. This result clearly demonstrated that D2 is important for the regulation of the PTM.

The present invention provided a method for promoting gonadal growth in an animal, comprising administration of thyroid hormone or its derivatives having thyroid hormone-like activity to the animal. Furthermore, this invention also provided a method for promoting gonadal growth in an animal, comprising introduction of a gene encoding type II deiodinase into the animal, and a transformed animal introduced with a gene encoding type II deiodinase into the animal. The method of the present invention provides a new method for promoting gonadal growth in an animal, through elucidation on the molecular mechanism of photoperiodism (photoperiodic time measurement) in birds.

## Claims

1. A method for promoting gonadal growth in an animal, comprising administration of thyroid hormone or its derivatives having thyroid hormone-like activity to the animal.

2. The method according to Claim 1, wherein said thyroid hormone is triiodothyronine.

3. The method according to Claim 1, wherein said animal is a bird or a mammal.

4. The method according to Claim 3, wherein said animal is a bird.

5. A method for promoting gonadal growth in an animal, comprising introduction of a gene encoding type II deiodinase into the animal

6. The method according to Claim 5, wherein said animal is a bird or a mammal.

7. The method according to Claim 6, wherein said animal is a bird.

8. A transformed animal introduced with a gene encoding type II deiodinase into the animal

9. The method according to Claim 8, wherein said animal is a bird or a mammal.

10. The method according to Claim 9, wherein said animal is a bird.
